# EUROPEAN PATENT APPLICATION

(11) **EP 3 998 044 A1**
(43) Date of publication of application: **18.05.2022**
(21) Application number: 21839308.0
(22) Date of filing: 18.01.2021
(51) Int. Cl.: A61F 2/24

(54) **SELF-EXPANDING ATRIOVENTRICULAR VALVE PROSTHESIS DEVICE**

(30) Priority: 15.09.2020 CN 202010967051; 15.09.2020 CN 202022015511 U
(71) Applicant: Jiangsu Trulive Medtech Co., Ltd, Rudong County Nantong Jiangsu 226400 (CN)
(72) Inventor: The designation of the inventor has not yet been filed
(74) Representative: Argyma
(86) International application number: PCT/CN2021/072488
(87) International publication number: WO 2022/057176

(57) **Abstract**

Disclosed is a self-expandable atrioventricular valve prosthesis device, including a main body portion of a frame structure, wherein the main body portion is implanted at a primary annulus of a heart and the main body portion includes an inflow segment, an outflow segment and a transition segment, wherein the inflow segment is located at an atrium end, the outflow segment is located at a ventricle end, and the transition segment is located between the inflow segment and the outflow segment. The device further includes at least one prosthetic valve leaflet fixed to the transition segment of the main body portion, and the prosthetic valve leaflet is configured with a connecting portion. The device further includes a chordae tendineae portion with one end fixed and the other end connected to the prosthetic valve leaflet via the connecting portion. In the present invention, by limiting the movement range of the prosthetic valve leaflet via the chordae tendineae portion, and by preventing the shear stress and the stress concentration on the valve leaflets exerted by the chordae tendineae portion via the connecting portion, the endurance of the prosthetic valve leaflet is increased.

## Description

### Field of the Invention

The present invention relates to the technical field of medical instruments, and more specifically, to a self-expandable atrioventricular valve prosthesis device implanted into a heart for replacing a primary valve.

### Description of the Prior Art

The heart contains four cavities, i.e., a right atrium (RA), a right ventricle (RV), a left atrium (LA), and a left ventricle (LV). During each cardiac cycle, pumping actions of the left and right sides of the heart generally occurs simultaneously. A valve separating the atrium from the ventricle is called an atrioventricular valve, and the atrioventricular valve functions as a one-way valve to ensure the normal flow of blood in the heart cavity. The atrioventricular valve between the left atrium and the left ventricle is a mitral valve, and the atrioventricular valve between the right atrium and the right ventricle is a tricuspid valve. A pulmonary valve directs the blood flow to a pulmonary artery, and to flow to a lung from there; the blood returns to the left atrium through pulmonary veins. An aortic valve directs the blood flow to pass through the arota and to flow to the peripheries from there. Normally, there is no direct connection between the ventricles or the atriums.

When the ventricle begins to be filled (dilated), the aortic and pulmonary valves close to prevent regurgitation from the arteries into the ventricle. Shortly thereafter, the atrioventricular valves open to allow unobstructed flow from the atrium into the corresponding ventricle. Shortly after the ventricular systole (i.e., ventricular evacuation), the tricuspid valve and the mitral valve normally close, thereby forming a seal for preventing the regurgitation from the ventricle to the corresponding atrium.

When there is a problem with the atrioventricular valve, it does not function properly, resulting in improper closure. The atrioventricular valve is a complex structure, typically including an annulus, valve leaflets, a chordae tendineae, and a support structure. Each of the atriums is connected to the valve thereof through an atrium vestibule. The mitral valve has two valve leaflets, the tricuspid valve has a similar structure with three valve leaflets, and the attachment or engagement between the corresponding surfaces of each of the valve leaflets helps to provide closure or seal for the valve, thereby preventing the blood from flowing in a wrong direction. During the ventricular systole, the valve leaflets being unable to be sealed is called poor engagement, which causes the blood to flow reversely (regurgitation) through the valve. The heart valve regurgitation may induce serious consequences for patients, which usually lead to heart failure, reduction of blood flow, reduction of blood pressure and/or reduction of the flow of oxygen to human tissues. The mitral valve regurgitation can further causes the blood flow back to the pulmonary veins from the left atrium, thereby resulting in hyperaemia. Severe valve regurgitation, if not treated, may cause permanent disability or death.

In recent years, there have been some breakthroughs in the field of prosthetic valves, but the mitral valve therapy still faces enormous challenges due to the complexity of the mitral valve and surrounding structures. For example, the challenges are the following: 1. How to solve the problems of valve leaflet prolapse and outward turn: after the prosthetic valve prosthesis is implanted, the risk of prolapse and outward turn for the valve leaflet exists due to excessive pressure of the ventricle when the heart compresses. 2. How to reduce the valve leaflet stress and increase the endurance of the prosthetic valve. The tricuspid valve also have the above problems.

### SUMMARY OF THE INVENTION

The present invention provides a self-expandable atrioventricular valve prosthesis device, which may solve the above drawbacks in the prior art.

The technical solution of the present invention is as follows:
A self-expandable atrioventricular valve prosthesis device includes a main body portion of a frame structure, wherein the main body portion is implanted at a primary annulus of a heart and the main body portion includes an inflow segment, an outflow segment and a transition segment, wherein the inflow segment is located at an atrium end, the outflow segment is located at a ventricle end, and the transition segment is located between the inflow segment and the outflow segment. The device further includes at least one prosthetic valve leaflet fixed to the transition segment of the main body portion, and the prosthetic valve leaflet is configured with a connecting portion. The device further includes at least one chordae tendineae portion with one end fixed and the other end connected to the prosthetic valve leaflet via the connecting portion; the chordae tendineae portion is used to limit the movement range of the prosthetic valve leaflet, thereby increasing the endurance of the prosthetic valve leaflet.

When the heart compresses, the risk of prolapse and outward turn for the prosthetic valve leaflet exists due to excessive pressure of the ventricle. In the present invention, starting from reducing the stress of the prosthetic valve leaflet and increasing the endurance of the valve leaflets, through the provision of the chordae tendineae portion connected to the valve leaflets, when the prosthetic valve leaflet is going to be subjected to prolapse and outward turn, the chordae tendineae portion generates a pulling force for the valve leaflets so that the valve leaflets recover to a normally-closed state, thereby enabling the valve leaflets to normally close the blood channel in the closed state, i.e., enabling the chordae tendineae portion here to function as a chordae tendineae. Considering that the tearing force and the stress of the valve leaflets may concentrate and the structure of the valve leaflets may be damaged over time when the chordae tendineae portion is directly connected to the valve leaflets, so the valve leaflets are provided with the connecting portion so that the acting force generated by the chordae tendineae portion is exerted onto the valve leaflets through the connecting portion by the indirect connection between the connecting portion and the valve leaflets, thereby reducing the stress of the valve leaflets and increasing the endurance of the valve leaflets.

In some embodiments, the connecting portion is configured on a surface of the prosthetic valve leaflet and located on a side close to the ventricle end, and the connecting portion configured on the surface may increase the contact area with valve leaflets, thereby reducing the stress concentration. In some embodiments, the connecting portion is configured at an edge of the prosthetic valve leaflet; and the connecting portion configured at the edge of the prosthetic valve leaflet may reduce the acting force exerted by the chordae tendineae portion, thereby reducing the stress. In some embodiments, there are a plurality of connecting portions, which are configured on the surface of the valve leaflets and the edge of the valve leaflets respectively, and the number of the connecting portions may be set according to actual clinical needs.

In some embodiments, the prosthetic valve leaflet includes a plurality of free edges, and an arc length of a portion where the connecting portion is connected to any one of the free edges accounts for 1/12 to 1/3 of an arc length of the free edge where the connecting portion is located. The longer the arc length of the portion where the connecting portion is connected to the free edge is, the smaller the movement range of the valve leaflets is; when the movement range is too small, an effective opening area of the valve leaflets may be affected; contrarily, the shorter the arc length of the portion where the connecting portion is connected to the free edge is, the higher the risk of friction between the valve leaflets and the main body portion is.

In some embodiments, a pre-set angle α is configured between the connecting portion and the prosthetic valve leaflets, wherein α is greater than 0 but smaller than or equal to 180°. With this angle, the connecting portion may not interfere with the normal opening and closing of the prosthetic valve leaflets, and also may not block the outflow tract.

In some embodiments, the chordae tendineae portion includes a first connecting end and a second connecting end, the first connecting end is connected to the connecting portion, and the second connecting end is fixed to the main body portion or the second connecting end is fixed to a primary tissue. When the chordae tendineae portion is connected to the main body portion, the connecting portion, the chordae tendineae portion and the main body portion may be implanted integrally, thereby simplifying the design of the transporting system. When the chordae tendineae portion is connected to the tissue, the outflow tract is prevented from being blocked by the connecting portion and the chordae tendineae portion, and also the angle α is designed to be greater as compared to the situation where the connecting portion is connected to the chordae tendineae portion so that the configuration of the included angle between the connecting portion and the surface of the valve leaflets is more flexible.

In some embodiments, the second connecting end is fixed to the inflow segment or the outflow segment. Preferably, the second connecting end is fixed to the outflow segment as a distance between the outflow segment and the connecting portion is small; therefore, the amount of the material used for the chordae tendineae portion is reduced; in addition, compared with being fixed to the inflow segment, fixing the second connecting end to the outflow segment may prevent the chordae tendineae portion from being entangled with the main body portion.

In some embodiments, the second connecting end is fixed to an upper tissue or a lower tissue, and preferably fixed to the lower tissue.

In some embodiments, when the second connecting end is connected to the tissue, the second connecting end of the chordae tendineae portion is configured to have the same shape as the corresponding connecting portion. When the second connecting end connected to the tissue has the same shape as the connecting portion, the chordae tendineae portion and the connecting portion may be forced evenly, and then the endurance of the chordae tendineae portion and the connecting portion may be guaranteed to a certain extend.

In some embodiments, when the prosthetic valve leaflet is in a closed state, the chordae tendineae portion is configured to be on the same straight line as the connecting portion. Then, the acting force of the connecting portion exerted by the chordae tendineae portion is the smallest, thereby enhancing the endurance of the connecting portion.

In some embodiments, a position close to a center of the connecting portion is further provided with a connecting hole, and the chordae tendineae portion is connected to the connecting portion by the connecting hole, so that the connection between the chordae tendineae portion and the connecting portion is simpler.

Further, in some embodiments, a periphery of the connecting hole is covered by a protective layer to buffer a pulling force generated by the chordae tendineae portion on the connecting portion.

In some embodiments, the self-expandable atrioventricular valve prosthesis device further includes at least one clamping member that enables a connecting action. The clamping member may be connected to a portion to be connected by way of at least one of stitching, riveting connection and embedding, preferably by way of stitching.

In some embodiments, when the number of the prosthetic valve leaflets is configured to be at least two, the clamping member is clamped on the prosthetic valve leaflets for connecting two adjacent prosthetic valve leaves to reduce the movement range of the prosthetic valve leaflets and further to avoid the friction between the movable prosthetic valve leaflets and the main body portion, thereby improving the endurance of the prosthetic valve leaflets. After the movement range of the prosthetic valve leaflets is reduced, correspondingly the movement range of the connecting portion and the chordae tendineae portion is also be limited, thereby further improving the endurance of the chordae tendineae portion and the connecting portion.

In some embodiments, the connecting portion may be selectively prepared integrally with the prosthetic valve leaflet and/or the chordae tendineae portion, e.g., the connecting portion is prepared integrally with the prosthetic valve leaflet and then connected to the chordae tendineae portion, or the connecting portion is prepared integrally with the chordae tendineae portion and then connected to the prosthetic valve leaflet, or the prosthetic valve leaflet, the connecting portion and the chordae tendineae portion are prepared integrally. The chordae tendineae portion may be molded into a linear structure and a sheet-like structure, and the chordae tendineae portion may also be molded into a spiral structure, thereby providing a certain flexibility

In some embodiments, the prosthetic valve leaflet, the connecting portion, and the chordae tendineae portion are prepared respectively and are then connected with each other as a whole. The molding way for the prosthetic valve leaflet, the connecting portion, and the chordae tendineae portion may be selected according to actual clinical needs.

Compared with the existing technology, the present invention has the following beneficial effects:
1. For the atrioventricular valve prosthesis device of the present invention, connecting the prosthetic valve leaflet with the chordae tendineae portion limits the movement range of the valve leaflets, thereby preventing the prolapse and outward turn for the prosthetic valve leaflet; further, by adding the connecting portion on the valve leaflets, the direct connection between the prosthetic valve leaflet and the chordae tendineae portion is avoided to relieve the tearing force and the stress of the valve leaflets, thereby increasing the endurance of the valve leaflets; moreover, in the present invention, the movement range of the valve leaflets may be limited by the chordae tendineae portion, and the collision between the valve leaflets and the main body portion is prevented, thereby increasing the endurance of the valve leaflets.
2. For the atrioventricular valve prosthesis device of the present invention, with the configuration of the arc length of the portion where the connecting portion is connected to the free edge and the connection between the two adjacent valve leaflets realized by the clamping member, the movement range of the valve leaflets is further limited to avoid the collision between the valve leaflets and the main body portion while reducing the magnitude of movement of the connecting portion, and the movement range of the chordae tendineae portion is further reduced, thereby increasing the endurance of the chordae tendineae portion.

### BRIEF DESCRIPTION OF THE DRAWINGS

Fig. 1 is a structural schematic diagram after the atrioventricular valve prosthesis device is implanted according to the present invention;
Fig. 2 is a structural schematic diagram of a main body portion according to the present invention;
Fig. 3 is a partial structural schematic diagram of a prosthetic valve leaflet according to Embodiment 1 of the present invention;
Fig. 4 is a partial structural schematic diagram of another prosthetic valve leaflet according to Embodiment 1 of the present invention;
Fig. 5 is a partial structural schematic diagram of the atrioventricular valve prosthesis device according to Embodiment 1 of the present invention;
Fig. 6 is a partial structural schematic diagram of another atrioventricular valve prosthesis device according to Embodiment 1 of the present invention;
Fig. 7 is a local structural schematic diagram of a prosthetic valve leaflet in an opening state according to Embodiment 1 of the present invention;
Fig. 8 is a local structural schematic diagram of a prosthetic valve leaflet in a closing state according to Embodiment 1 of the present invention;
Fig. 9 is a local structural schematic diagram of another atrioventricular valve prosthesis device according to Embodiment 1 of the present invention;
Fig. 10 is a local structural schematic diagram of the atrioventricular valve prosthesis device according to Embodiment 2 of the present invention;
Fig. 11 is another local structural schematic diagram of the atrioventricular valve prosthesis device according to Embodiment 2 of the present invention;
Fig. 12 is a local structural schematic diagram of the atrioventricular valve prosthesis device according to Embodiment 3 of the present invention;
Fig. 13 is a local structural schematic diagram of another atrioventricular valve prosthesis device according to Embodiment 3 of the present invention;
Fig. 14 is a three-dimensional schematic diagram of a clamping member according to Embodiment 4 of the present invention;
Fig. 15 is a structural schematic diagram of connecting the prosthetic valve leaflet by way of stitching according to Embodiment 4 of the present invention;
Fig. 16 is a structural schematic diagram of the prosthetic valve leaflet and the clamping member according to Embodiment 4 of the present invention.

References for numerals: atrioventricular valve prosthesis device-100; first region-101; second region-102; third region-103; main body portion-110; prosthetic valve leaflet-130; connecting portion-140; chordae tendineae portion-150; inflow segment-111; transition segment-112; outflow segment-113.

### DESCRIPTION OF THE PREFERRED EMBODIMENTS

The present invention provides a self-expandable atrioventricular valve prosthesis device including a main body portion, a prosthetic valve leaflet and a chordae tendineae portion, which is used to replace a diseased primary valve, and has functions of opening or closing the blood channel. The atrioventricular valve prosthesis device may be designed to be a mitral valve prosthesis for replacing a diseased mitral valve, or may be designed to be a tricuspid valve prosthesis to replace a diseased tricuspid valve. The present invention will be further described with the mitral valve as an example.

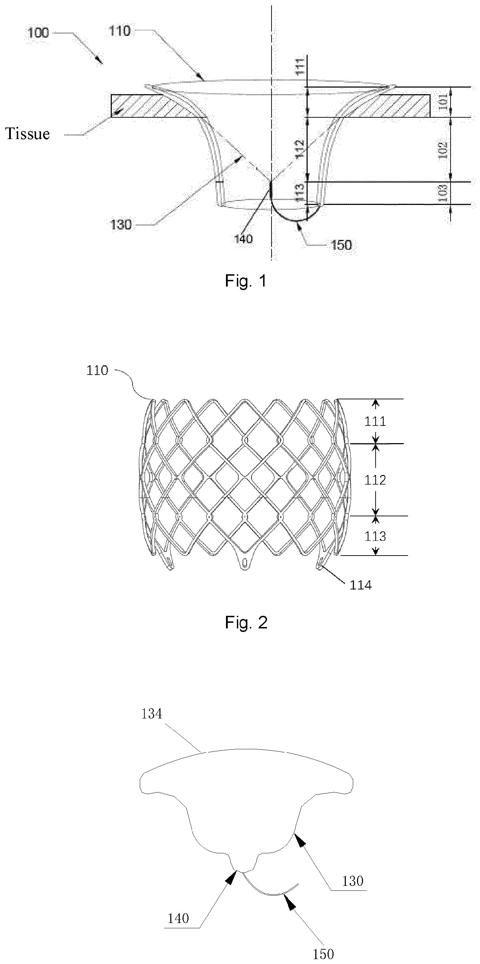

As shown in Fig. 1, the atrioventricular valve prosthesis device 100 is the mitral valve prosthesis, which may be divided longitudinally into a first region 101, a second region 102, and a third region 103. After the mitral valve prosthesis is implanted into a human body, the first region 101 is attached to a primary mitral valve annulus of the heart to prevent the prosthesis valve from falling from the left atrium into the left ventricle, and the second region 102 is used to carry the prosthetic valve leaflet 130 while being supported on the tissue by the anchoring force of the main body portion for functioning as an anchor and a seal. Optionally, the mitral valve prosthesis may only replace an anterior valve leaflet or a posterior valve leaflet of the mitral valve, i.e., half the prosthetic valve prosthesis is used in cooperation with half the primary valve leaflet.

Corresponding to the valve prosthesis, with reference to Fig. 2, the main body portion 110 is divided into an inflow segment 111, a transition segment 112, and an outflow segment 113, so as to provide the valve prosthesis with several functions, which include functioning as a main body structure and an anchoring structure (including an anchoring claw structure grabbing the valve leaflets or puncturing into the valve leaflets, and so on), as a support member carrying the internal prosthetic valve leaflet 130, a connecting portion 140 and the chordae tendineae portion 150, as a sealing member that inhibits the valve peripheral leakage between the valve prosthesis and the primary valve and as a structure (a hanging tab or a fixing tab 114) connected to a transporting system, and so on.

The main body portion 110 is of a frame structure with mesh holes, which is formed by weaving or cutting metal materials. If the valve prosthesis adopts is implanted by a method of intervening a catheter, the main body portion 110 is made from a nickel-titanium alloy or other biocompatible materials with shape memory characteristics, or may be made from an elastically deformable material or a plastically deformable material such as a balloon-expandable material. After being implanted, the main body portion 110 may be self-expanded into a pre-set structure, thereby being anchored at the primary annulus.

The prosthetic valve leaflet 130 dynamically switches between the opening state and a closing state. When the prosthetic valve leaflet is in the closing state, the prosthetic valve leaflet 130 is closed or converges by way of sealing and bonding, and the number of the prosthetic valve leaflets may be the same as or different from that of the primary valve leaflet. The prosthetic valve leaflet 130 may be formed by any suitable materials or a combination of materials. The prosthetic valve leaflet may be prepared by biological tissues such as chemically stable tissues from the heart valves of animals such as pigs. The prosthetic valve leaflet may also be prepared by pericardial tissues such as cattle (bovine pericardial tissues), sheep (ovine pericardial tissues), pigs (porcine pericardial tissues), or horses (equine pericardial tissues). The prosthetic valve leaflet may also be prepared by small intestinal submucosa tissues. In addition, synthetic materials may also be selected, such as expanded PTFE or polyester. Optionally, the material further includes thermoplastic polyurethane polyester, polyether urethane, segmented polyether urethane, silicone polyether urethane, silicone-polyurethane polyester, and ultra-high molecular weight polyethylene. Moreover, a biocompatible polymer may further be included, which may optionally include polyolefin, elastomer, polyethylene glycol, polyethersulfone, polysulfone, polyvinylpyrrolidone, polyvinyl chloride, other fluorine-containing polymers, silicone polyester, siloxane polymers and/or olimers, and/or polycaprolactone, and block copolymers thereof. Optionally, the valve leaflet 130 has a surface treated by (or reacted with) anticoagulants, and the anticoagulant includes but is not limited to heparinized polymers.

In the present invention, with the acting force exerted on the valve leaflet 130 by the chordae tendineae portion 150, a movement range of the prosthetic valve leaflet 130 may be limited, thereby preventing the prolapse and outward turn of the prosthetic valve leaflet 130.

In the description of the present invention, it should be noted that "valve leaflet" and "prosthetic valve leaflet" have the same meaning. The term "atrium end" refers to an end portion close the atrium, and the term "ventricle end" refers to an end portion close to the ventricle.

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned device or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of terms "install", "connected with", and "connected to" should be understood in a broad sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. As used in the specification, the term "or" is usually used to include the meaning of "and/or", unless otherwise expressly stated.

The following further describes the present invention in combination with specific embodiments.

### Embodiment 1

The present embodiment provides a self-expandable atrioventricular valve prosthesis device. With reference to Figs. 1 to 9, the atrioventricular valve prosthesis device includes a main body portion 110, at least one prosthetic valve leaflet 130, and a chordae tendineae portion 150. The main body portion is of a frame structure and implanted at a primary annulus of a heart, and the main body portion includes an inflow segment, an outflow segment, and a transition segment. The inflow segment is located at an atrium end, the outflow segment is located at a ventricle end, and the transition segment is located between the inflow segment and the outflow segment. The prosthetic valve leaflet 130 is fixed to the transition segment of the main body portion, and a connecting portion 140 is configured on a position close to the edge of the prosthetic valve leaflet 130. The chordae tendineae portion has one end fixed and the other end connected to the prosthetic valve leaflet 130 via the connecting portion 140 to limit the movement range of the prosthetic valve leaflet 130, thereby increasing the endurance of the prosthetic valve leaflet 130.

When the heart compresses, the risk of prolapse and outward turn for the prosthetic valve leaflet 130 exists due to excessive pressure of the ventricle. In the present embodiment, to reduce the stress of the prosthetic valve leaflet 130 and increase the endurance of the valve leaflet 130, a chordae tendineae portion is connected to the valve leaflet 130. When the prosthetic valve leaflet 130 is about to prolapse and outward-turn, the chordae tendineae portion generates a pulling force for the valve leaflet 130 so that the valve leaflet 130 recovers to a normally-closed state, thereby enabling the valve leaflet 130 to close the blood channel in the closing state normally. In other words, in the present embodiment, the chordae tendineae portion 150 functions as a chordae tendineae. Considering that the tearing force and the stress of the valve leaflet 130 may concentrate and the structure of the valve leaflet 130 may be damaged over time when the chordae tendineae portion 150 is directly connected to the valve leaflet 130. Therefore, in the present embodiment, the valve leaflet 130 is provided with the connecting portion 140 so that the chordae tendineae portion 150 is indirectly connected to the valve leaflet 130 via the connecting portion 140, and the acting force generated by the chordae tendineae portion 150 is exerted onto the valve leaflet 130 via the connecting portion 140, thereby reducing the stress of the valve leaflet 130 and increasing the endurance of the valve leaflet 130.

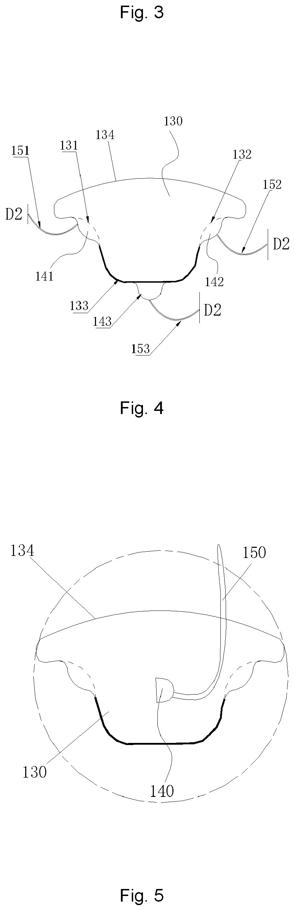

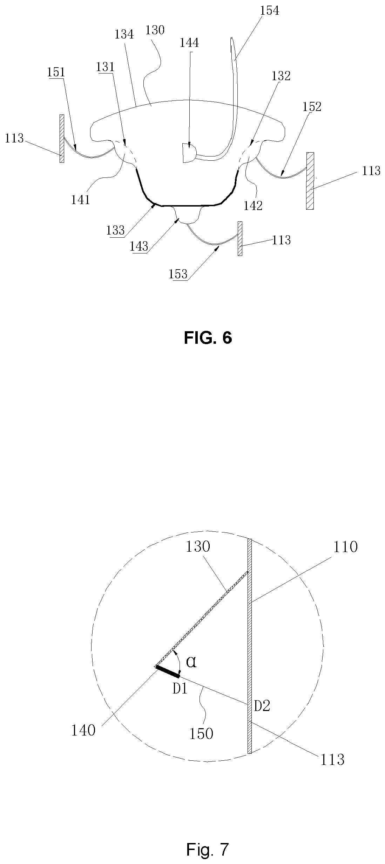

Specifically, the connecting portion 140 may be configured on the edge of the prosthetic valve leaflet 130. With reference to Fig. 3, the prosthetic valve leaflet is provided with a fixing edge 134 and is fixed to the main body portion 110 via the fixing edge 134. The number of the connecting portion 140 is one, and the connecting portion 140 is configured at the edge of the valve leaflet. In Fig. 4, the number of the connecting portion is configured to be three, and the connecting portion located at the edge of the valve leaflets may reduce the acting force exerted by the chordae tendineae portion so as to reduce the stress. Naturally, the connecting portion 440 may also be configured on the surface of the prosthetic valve leaflet 130, as shown in Fig. 5, thereby increasing the contact area between the connecting portion 140 and the valve leaflet 130 and reducing the stress concentration. The connecting portion 140 is preferably configured on a surface of a side close to the ventricle end. Optionally, as shown in Fig. 6, the connecting portion 140 may also be configured at the edge of the prosthetic valve leaflet 130 and the surface thereof, respectively, and then there are a plurality of connecting portions 140, wherein each of the connecting portions is connected to one chordae tendineae portion 150 respectively.

The prosthetic valve leaflet 130 includes a plurality of free edges, and the connecting portion 140 may be located on any position of any one of the free edges, wherein an arc length of a portion where the connecting portion 140 is connected to any one of the free edges accounts for 1/12 to 1/3 of an arc length of the free edge where the connecting portion is located. The longer the arc length of the portion where the connecting portion 140 connected to the free edge is, the smaller a movement range of the prosthetic valve leaflet 130 is; when the movement range is too small, an effective opening area of the prosthetic valve leaflet 130 may be affected. Contrarily, the shorter the arc length of the portion where the connecting portion 140 connected to the free edge is, the higher the risk of friction between the prosthetic valve leaflet 130 and the main body portion 110 is.

Specifically, continuously referring to Fig. 6, in the present embodiment, the prosthetic valve leaflet 130 includes a first free edge 131, a second free edge 132, a third free edge 133, and a fixing edge 134. The fixing edge 134 is fixed to the transition segment 112 of the main body portion 110; the third free edge 133 is located between the first free edge 131 and the second free edge 132, and the first free edge 131, the second free edge 132 and the third free edge 133 together form the edge of the prosthetic valve leaflet 130. The first free edge 131 is configured with a first connecting portion 141, the second free edge 132 is configured with a second connecting portion 142, the third free edge 133 is configured with a third connecting portion 143, and the surface of the prosthetic valve leaflet 130 is configured with a fourth connecting portion 144.

Preferably, the third connecting portion 143 is located at a center of the third free edge 133, such that the force exerted on left and right sides of the third connecting portion 143 by the chordae tendineae portion is uniform when the heart dilates, thereby improving the endurance of the third connecting portion 143. At the same time, when the heart compresses, the third connecting portion 143 enables the prosthetic valve leaflet 130 to avoid the prolapse and outward turn. Naturally, in other alternative embodiments, the connecting portion may be located on any one of, any two of, or any three of the first free edge 131, the second free edge 132, the third free edge 133, and the surface of the valve leaflet 130, and the number and arrangement position of the connecting portion 140 may be selected according to actual clinical needs, which is not limited here.

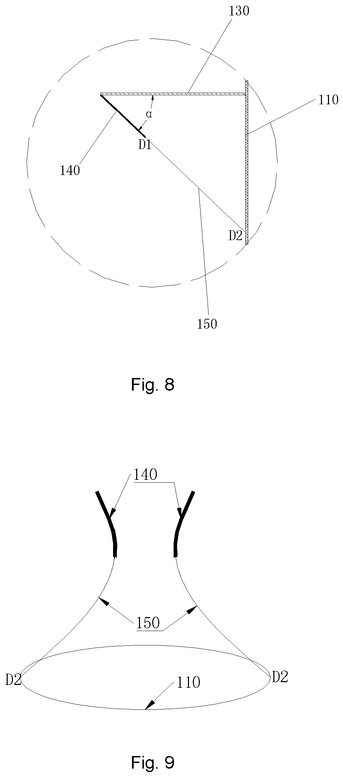

With reference to Figs. 7 and 8, partial structural schematic diagrams of the prosthetic valve leaflet 130, the connecting portion 140, the chordae tendineae portion 150, and the main body portion 110 are shown respectively. In the present embodiment, a pre-set angle α is configured between the connecting portion 140 and the prosthetic valve leaflet 130, wherein α is greater than 0 but less than or equal to 180°; with this angle, the connecting portion 140 may not interfere with the normal opening and closing of the prosthetic valve leaflet 130, and also may not block the outflow tract. In Fig. 7, the prosthetic valve leaflet 130 is in the opening state; in Fig. 8, the prosthetic valve leaflet 130 is in the closing state.

The connecting portion 140 may have a shape including at least one of a triangle, a quadrangle, a pentagon, a circle-like shape and a shape with a sleek contour, wherein the quadrangle includes a squares, a rectangle, a diamond, a trapezoid, etc.; the circle-like shape is a positive polygon with no less than six edges; the shape with a sleek contour includes a circle, an egg circle, a semi-circle, a semi-egg circle, a petal shape, etc. In the present embodiment, preferably, the connecting portion has the shape with a sleek contour, with reference to Figs. 3 to 6, and the connecting portion 140 is the semi-circle, which is more conducive to the dispersion of the stress.

Further, continuously referring to Figs. 6 to 8, the chordae tendineae portion 150 includes a first connecting end D1 and a second connecting end D2. The first connecting end D1 is connected to the connecting portion 140, and the second connecting end D2 is fixed to the main body portion 110. When the chordae tendineae portion 150 is connected to the main body portion 110, the connecting portion 140, the chordae tendineae portion 150, and the main body portion 110 may be implanted integrally, thereby simplifying the design of the transporting system.

In the present embodiment, the first connecting portion 141 is connected to a first chordae tendineae portion 151, the second connecting portion 142 is connected to a second chordae tendineae portion 152, the third connecting portion 143 is connected to a third chordae tendineae portion 153, and the fourth connecting portion 143 is connected to a fourth chordae tendineae portion 154. The first chordae tendineae portion 151, the second chordae tendineae portion 152, the third chordae tendineae portion 153, and the fourth chordae tendineae portion 154 are fixed to a pre-set position of the outflow segment 113, respectively. Naturally, in other alternative embodiments, the first chordae tendineae portion 151, the second chordae tendineae portion 152, the third chordae tendineae portion 153, and the fourth chordae tendineae portion 154 may be independently fixed to the inflow segment 111. When the chordae tendineae portion 150 is fixed to the inflow segment 111, the second connecting end D2 of the chordae tendineae portion 150 extends to the inflow segment 111 from the mesh holes of the frame structure or from the outflow segment 113, and then is fixed. Compared with being fixed to the inflow segment 111, the amount of the material used is reduced as the distance between the connecting portion 140 and the outflow segment 113 is shorter; on the other hand, the connecting portion 140 is directly fixed to the outflow segment 113, so that the chordae tendineae portion 150 is prevented from being entangled with the frame structure of the main body portion.

Further, with reference to Fig. 9, a side of a position where two chordae tendineae portions 150 connected to the main body portion 110 may be bridge shape, umbrella shape, and cone shape, etc., preferably the cone shape. Preferably, the second connecting end D2 of the chordae tendineae portion 150 covers the main body portion 110, such that the force applied to the chordae tendineae portion 150 may be evenly dispersed on the main body portion 110 when the heart compresses, thereby ensuring the endurance of the chordae tendineae portion 150.

In a preferred embodiment, the chordae tendineae portion 150 is configured to be on the same straight line as the connecting portion 140 when the prosthetic valve leaflet 130 is in the closing state, as shown in Figs. 7 and 8, and then the acting force of the connecting portion 140 exerted by the chordae tendineae portion 150 is the smallest, thereby enhancing the endurance of the connecting portion 140.

Further, the connecting portion 140 may be prepared integrally with the prosthetic valve leaflet 130 and then is connected to the chordae tendineae portion 150; the connecting portion 140 may be prepared integrally with the chordae tendineae portion 150 and then is connected to the prosthetic valve leaflet 130; or, the prosthetic valve leaflet 130, the connecting portion 140, and the chordae tendineae portion 150 are prepared integrally as a whole. Naturally, the prosthetic valve leaflet 130, the connecting portion 140, and the chordae tendineae portion 150 may also be prepared respectively and then are combined with each other, wherein the way of the combination includes, but is not limited to stitching, glued connection, riveting connection, embedding and fixing with clamp tabs, and so on. The molding and connection ways of the prosthetic valve leaflet 130, the connecting portion 140, and the chordae tendineae portion 150 may be selected according to actual needs, which is not be repeated here.

In the present embodiment, the materials for the prosthetic valve leaflet 130, the connecting portion 140, and the chordae tendineae portion 150 may be the same or different from each other. Preferably, the chordae tendineae portion 150 may adopt a material with a certain flexibility, and molded into a linear structure and a sheet-like structure; or the chordae tendineae portion 150 is molded into a structure with a certain stiffness, such as a spiral shape, thereby providing a certain pulling force when the prolapse and outward-turn of the prosthetic valve leaflet 130 occur. The materials for the prosthetic valve leaflet 130, the connecting portion 140, and the chordae tendineae portion 150 are not used to limit the scope of the present invention, and may be selected according to actual clinical needs.

Specifically, the materials for the connecting portion 140 and the chordae tendineae portion 150 may be selected from biological tissues, synthetic materials, or biocompatible polymers. For example, the biological tissues may be prepared by the following: chemically stable tissues from the heart valves of animals such as pigs; or animal pericardial tissues such as cattle (bovine pericardial tissues), sheep (ovine pericardial tissues), pigs (porcine pericardial tissues), horses (equine pericardial tissues), or small intestinal submucosa tissues. For example, the synthetic materials include expanded PTFE or polyester, and optionally, further include thermoplastic polyurethane polyester, polyether urethane, segmented polyether urethane, silicone polyether urethane, silicone-polyurethane polyester, and ultra-high molecular weight polyethylene; the biocompatible polymers optionally include polyolefin, elastomer, polyethylene glycol, polyethersulfone, polysulfone, polyvinylpyrrolidone, polyvinyl chloride, other fluorine-containing polymers, silicone polyester, siloxane polymers and/or olimers, and/or polycaprolactone, and block copolymers thereof.

### Embodiment 2

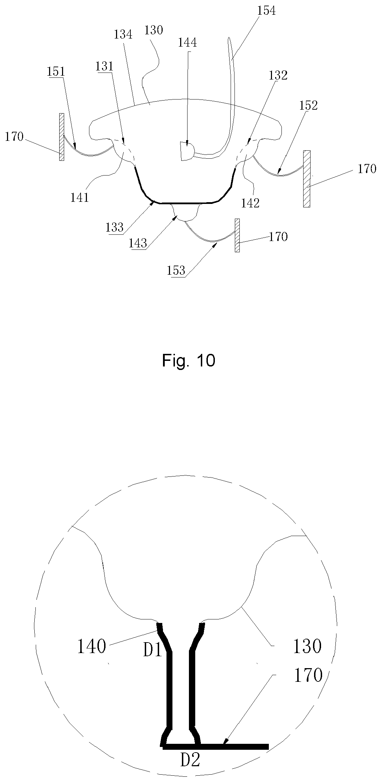

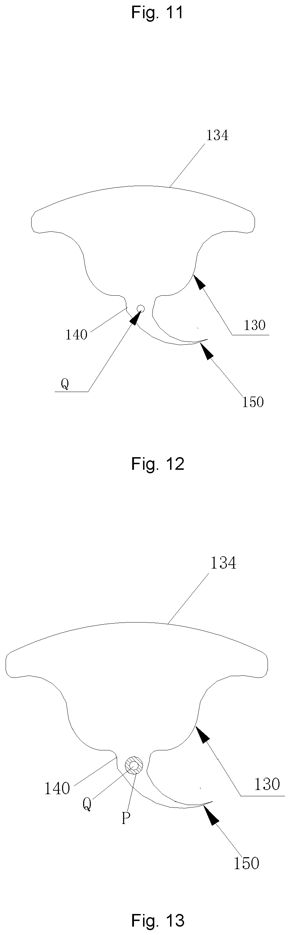

The present embodiment provides a self-expandable atrioventricular valve prosthesis device, with reference to Figs. 10 and 11, wherein the structure of the atrioventricular valve prosthesis device is similar to that of Embodiment 1 but differs from the latter in that the second connecting end D2 of the chordae tendineae portion 150 in the present embodiment is fixed to the primary tissue.

The primary tissue includes two parts, a tissue of the atrium end (upper tissue) and a tissue of the ventricle end (lower tissue). The lower tissue includes but is not limited to a papillary muscle, an apex cordis, an interventricular septum, and so on. When the chordae tendineae portion 150 is connected to a tissue 170, the outflow tract is prevented from being blocked by the connecting portion 140 and the chordae tendineae portion 150, and also the angle α is designed to be greater as compared to the situation where the connecting portion 140 is connected to the main body portion.

In the present embodiment, the second connecting end D2 of the chordae tendineae portion 150 is preferably fixed to the lower tissue 172 to prevent the second connecting end D2 from entangling with the main body portion 110 when the second connecting end passes through the frame.

When the heart compresses, to ensure the endurance of the chordae tendineae portion 150, the force applied to the chordae tendineae portion 150 is required to be dispersed evenly on the tissue 170. However, when the contact area between the second connecting end D2 of the chordae tendineae portion 150 and the tissue 170 is too large, the outflow tract of the blood may be easily interfered with, affecting the hemodynamics; when the contact area is too small, the force applied to the chordae tendineae portion 150 and the connecting portion 140 may be increased correspondingly. Therefore, preferably, a shape of the second connecting end D2 of the chordae tendineae portion 150 is the same as that of the corresponding connecting portion 140. When the second connecting end D2 connected to the tissue 170 has the same shape as the connecting portion 140, the chordae tendineae portion 150 and the connecting portion 140 may be forced evenly, and then the endurance of the chordae tendineae portion 150 and the connecting portion 140 may be guaranteed to a certain extend.

### Embodiment 3

The present embodiment provides a self-expandable atrioventricular valve prosthesis device, which is an improvement made based on Embodiment 1 or 2, wherein the connecting portion 140 is further provided with a connecting hole Q, with reference to Fig. 12, and the chordae tendineae portion 150 is connected to the connecting portion 140 via the connecting hole Q. The connecting hole Q may be located on any position of the connecting portion 140, preferably on the center of the connecting portion 140, so as to disperse the stress evenly. The number of the connecting hole Q is at least 1, and the shape thereof may be a rectangle, a circle, a triangle and so on, preferably the circle, as the circle may enable the pulling force applied to the connecting portion 140 to be dispersed evenly on the connecting portion 140 and the chordae tendineae portion 150.

Further, a periphery of the connecting hole Q is covered with a protective layer P. With reference to Fig. 13, the protective layer P may be a ring of materials with better biocompatibility covered on the periphery of the connecting hole Q such as metal sheets, PTFE membrane sheets, and polyesters. The protective layer P is used to buffer the pulling force of the connecting portion 140 exerted by the chordae tendineae portion 150 when the prosthetic valve leaflet 130 moves, so as to increase the endurance of the connecting portion 140.

### Embodiment 4

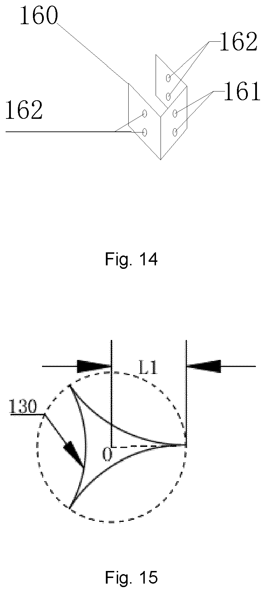

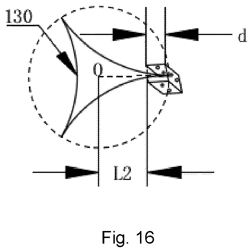

The present embodiment provides a self-expandable atrioventricular valve prosthesis device, which is an improvement made based on Embodiments 1, 2, or 3, wherein the atrioventricular valve prosthesis device further includes at least one clamping member 160 for realizing the connection, as shown in Figs. 14 and 16.

The clamping member 160 may be connected to a portion to be connected by way of at least one of stitching, riveting connection, and embedding, preferably by way of stitching. For example, the clamping member 160 may realize the connection between the connecting portion 140 and the prosthetic valve leaflet 130, and the connection between the connecting portion 140 and the chordae tendineae portion 150.

In an embodiment, when the number of the prosthetic valve leaflet 130 is configured to be at least two, two adjacent prosthetic leaflets 130 are connected with each other by clamping the clamping member 160 on the prosthetic valve leaflets 130, so as to reduce the movement range of the prosthetic valve leaflets 130.

Specifically, the clamping member 160 is a clip. The clip includes a valve leaflet stitching portion 162 and a main body portion stitching portion 161. The clip has a width d. The free edges of the two adjacent prosthetic valve leaflets are partially clamped by the clip. The clip is fixed to the prosthetic valve leaflet 130 via the valve leaflet stitching portion 162, and the clip is fixed to the main body portion 110 via the main body portion stitching portion 161, thereby realizing the fixation of the prosthetic valve leaflet 130.

Fig. 15 shows connecting the prosthetic valve leaflets 130 by way of stitching, and L1 is a distance between a stitching point and a closing position O of the prosthetic valve leaflet; Fig. 16 shows connecting the prosthetic valve leaflet 130 by way of stitching with clips, L2 is a distance between one end of the clip far away from the main body portion and a closing position O of the prosthetic valve leaflet. L1=L2+d, i.e., L1>L2; therefore, the movement range of the prosthetic valve leaflet 130 in Fig. 16 is smaller than the magnitude of movement of the prosthetic valve leaflet 130 in Fig. 15. When stitched by the clips, the movement range of the prosthetic valve leaflet 130 is reduced, such that the friction between the movable prosthetic valve leaflet 130 and the main body portion is avoided, thereby improving the endurance of the prosthetic valve leaflet 130. After the movement range of the prosthetic valve leaflet 130 is reduced, correspondingly the movement ranges of the connecting portion 140 and the chordae tendineae portion 150 are also be limited, thereby further improving the endurance of the chordae tendineae portion 150 and the connecting portion 140.

The materials for the clamping member 160 may be at least one of the materials with better biocompatibility, such as metals, pericardial tissues and PTFE, and so on.

The above disclosure is only the preferred embodiment of the present invention. The preferred embodiments do not describe all the details, and are not intended to limit the invention only to be the specific embodiments. It should be understood that these embodiments are used only to illustrate the present invention and not to limit the scope of protection of the present invention.

In practical application, the improvement and adjustment made by those skilled in the art according to the present invention still belong to the scope of protection of the present invention. It is obvious that various modifications and changes can be made to the content of the specification. With the purpose of better explaining the principle and practical use of the present invention, the present invention selects and specifically describes the embodiments, such that a person skilled in the art can well utilize the present invention. The present invention is merely limited by the appended claims and the scope and equivalents thereof.

## Claims

1. A self-expandable atrioventricular valve prosthesis device, comprising:
a main body portion of a frame structure, wherein the main body portion is implanted at a primary annulus of a heart, and the main body portion including an inflow segment, an outflow segment, and a transition segment, wherein the inflow segment is located at an atrium end, the outflow segment is located at a ventricle end, and the transition segment is located between the inflow segment and the outflow segment;
at least one prosthetic valve leaflet, fixed to the transition segment of the main body portion, the prosthetic valve leaflet being configured with a connecting portion;
at least one chordae tendineae portion, with one end fixed and the other end connected to the prosthetic valve leaflet by the connecting portion; the chordae tendineae portion is used to limit the movement range of the prosthetic valve leaflet, thereby increasing the endurance of the prosthetic valve leaflet.

2. The self-expandable atrioventricular valve prosthesis device according to claim 1, wherein the connecting portion is configured on a surface of the prosthetic valve leaflet, or the connecting portion is configured at an edge of the prosthetic valve leaflet.

3. The self-expandable atrioventricular valve prosthesis device according to claim 1, wherein the prosthetic valve leaflet comprises a plurality of free edges, and an arc length of a portion where the connecting portion is connected to any one of the free edges accounts for 1/12 to 1/3 of an arc length of the free edge where the connecting portion is located.

4. The self-expandable atrioventricular valve prosthesis device according to claim 1, wherein a pre-set angle α is configured between the connecting portion and the prosthetic valve leaflet, wherein α is greater than 0 but smaller than or equal to 180°.

5. The self-expandable atrioventricular valve prosthesis device according to claim 1, wherein the chordae tendineae portion comprises a first connecting end and a second connecting end, the first connecting end is connected to the connecting portion, and the second connecting end is fixed to the main body portion or the second connecting end is fixed to a primary tissue.

6. The self-expandable atrioventricular valve prosthesis device according to claim 5, wherein the second connecting end is fixed to the inflow segment or the outflow segment.

7. The self-expandable atrioventricular valve prosthesis device according to claim 5, wherein the second connecting end is fixed to an upper tissue or a lower tissue.

8. The self-expandable atrioventricular valve prosthesis device according to claim 5, wherein when the second connecting end is connected to the tissue, the second connecting end of the chordae tendineae portion is configured to have a same shape as the corresponding connecting portion.

9. The self-expandable atrioventricular valve prosthesis device according to claim 1, wherein when the prosthetic valve leaflet is in a closed state, the chordae tendineae portion is configured to be on a same straight line as the connecting portion.

10. The self-expandable atrioventricular valve prosthesis device according to any one of claims 1 to 9, wherein a position close to a center of the connecting portion is further provided with a connecting hole, and the chordae tendineae portion is connected to the connecting portion by the connecting hole.

11. The self-expandable atrioventricular valve prosthesis device according to claim 10, wherein a periphery of the connecting hole is covered by a protective layer to buffer a pulling force generated by the chordae tendineae portion on the connecting portion.

12. The self-expandable atrioventricular valve prosthesis device according to any one of claims 1 to 9 or claim 11, further comprising at least one clamping member that enables a connecting action.

13. The self-expandable atrioventricular valve prosthesis device according to claim 12, wherein when the number of the prosthetic valve leaflets is configured to be at least two, the clamping member is clamped on the prosthetic valve leaflet for connecting two adjacent prosthetic valve leaves to reduce the movement range of the prosthetic valve leaflet.

14. The self-expandable atrioventricular valve prosthesis device according to any one of claims 1 to 9 or claim 11 or claim 13, wherein the connecting portion may be selectively prepared integrally with the prosthetic valve leaflet and/or the chordae tendineae portion, or the prosthetic valve leaflet, the connecting portion and the chordae tendineae portion are prepared respectively and are then connected with each other as a whole.
